Europäisches Patentamt

European Patent Office

Office européen des brevets

(11) Publication number: 0 488 152 A2

# EUROPEAN PATENT APPLICATION

(21) Application number: 91120157.2

(22) Date of filing: 26.11.91

(51) Int. Cl.5: G01N 33/543, G01N 33/58, G01N 21/64, //G01N33/574, G01N33/68, C12Q1/68

(30) Priority: 30.11.90 JP 339385/90
28.02.91 JP 34031/91

(43) Date of publication of application:
03.06.92 Bulletin 92/23

(84) Designated Contracting States:
DE GB

(71) Applicant: HITACHI, LTD.
6, Kanda Surugadai 4-chome
Chiyoda-ku, Tokyo 101(JP)

(72) Inventor: Imai, Kazumichi
Tsukubadai Apato 1-103, 663, Ichige
Katsuta-shi(JP)
Inventor: Nomura, Yasushi
991-15, Motoyoshidacho
Mito-shi(JP)
Inventor: Koga, Masataka
43-2, Taiseicho
Katsuta-shi(JP)
Inventor: Tokinaga, Daizo

42-10, Mejirodai-1-chome
Hachioji-shi(JP)
Inventor: Takahashi, Satoshi
Keimeiryo 209, 14-13, Honda-3-chome
Kokubunji-shi(JP)
Inventor: Oki, Hiroshi
8-1, Migimomi
Tsuchiura-shi(JP)
Inventor: Miyake, Ryo
Park de Kotten 139
Enschede(NL)
Inventor: Okano, Kazunori
17-2-402, Honcho-5-chome
Shiki-shi(JP)
Inventor: Yasuda, Kenji
52-5, Minamidai-4-chome
Nakano-ku, Tokyo(JP)

(74) Representative: Strehl, Schübel-Hopf,
Groening
Maximilianstrasse 54 Postfach 22 14 55
W-8000 München 22(DE)

(54) Method for immunoassay and apparatus therefor.

(57) A method for immunoassay of a trace vital component is provided. Using fine particles as label or marker 21, the fine particles are captured on reaction solid phase 10 in proportion to an amount of analyte by a specific reaction such as antigen-antibody reaction. Then, the fine particles are liberated and the number of fine particles is counted to determine the amount of analyte. The solution to be assayed containing the liberated matters is introduced into flow cell 1 and pulse-like fluorescence emitted when the fine particles pass through a flux of laser light irradiated from the direction crossing the flow at the right angle is detected and the pulse is counted to count the number of fine particles 21.

The marker or label, i.e., the fine particles once captured on the reaction solid phase are liberated and then counted. Therefore, influence of the label non-specifically bound to the solid phase can be eliminated. By using the fine particles as the label and counting the number of the particles, detection having a high linearity can be realized even at a low concentration.

FIG. 4

EP 0 488 152 A2

BACKGROUND OF THE INVENTION

Field of the Invention

The present invention relates to a method for immunoassay and an apparatus for immunoassay. More particularly, the present invention relates to a method for immunoassay which comprises labeling an analyte such as an antigen, an antibody or a hapten contained in a biological sample, introducing the labeled analyte to a flow cell and performing optical determination, and to an apparatus for the immunoassay.

Related Art Statement

For determining a trace substance in a biological sample such as blood, a method for immunoassay utilizing an antigen-antibody reaction is known. Japanese Patent Application KOKAI No. 1-187459 which is one of the prior art of this type discloses a method for immunoassay using an antigen-antibody reaction in combination with photoacoustic spectrometry.

That is, according to Japanese Patent Application KOKAI No. 1-187459, a sample containing an antigen as an analyte is charged in a reactor, onto the inside wall of which an antibody has been immobilized. Then, a suspension containing the liberated fine particles in the reactor is formed, via the steps of washing and removing the unreacted sample, adding a solution containing an antibody to which fine particles such as latex or an inorganic oxide has been bound, washing and removing an excess of the antibody and adding a liberating solution. The suspension is then transferred to a photoacoustic cell and exposed intermittently to a laser light to detect a change in pressure in the photoacoustic cell. A photoacoustic signal is thus obtained.

On the other hand, Japanese Patent Application KOKAI No. 63-214668 proposes a highly sensitive method for immunoassay. In the prior art, a labeled antibody obtained by binding microcapsules encapsulated with a fluorescent substance to an antibody is used. Any solid phase is not used in this prior art. The microcapsule-labeled antibody is added to a sample containing a cell to be assayed to effect immune binding. After washing by centrifugation, the suspension is charged in a sheath flow cell of flow cyto-meter. When the immune complex of the cell and the labeled antibody flows across a flux of laser light irradiated in the flow cell, fluorescence from the microcapsule is detected.

An example utilizing a sheath flow cell, though it is irrelevant to immunoassay, is disclosed in, e.g., Japanese Patent Application KOKAI No. 2-80937. According to the prior art, a sample container charged with stained blood cells is transferred to a sampling position and a blood cell suspension in the sample container is supplied to an inlet room of the sheath flow cell through a pipetting nozzle. The flow cell is so constructed that the flow cell is exposed to a laser light; when the stained cells move across a flux of the laser light, fluorescence and scattered light are detected.

In Japanese Patent Application KOKAI No. 1-187459 described above, a photoacoustic signal is detected and the signal obtained is dependent on the concentration of the whole suspension. Therefore, where an analyte has a very small concentration, it is difficult to determine the analyte with high precision. Where a labeled antibody is charged in a reactor with solid phase, the labeled antibody not only reacts specifically with the antigen bound to the solid phase but is also adsorbed non-specifically with the surface of the solid phase. Thus, this method involves a problem that the labeled antibody adsorbed non-specifically cannot be removed by ordinary washing operations. Where sodium hydroxide solution is used as a solution for liberating the marker as in Japanese Patent Application KOKAI No. 1-187459, however, both the specifically bound labeled particles and the non-specifically adsorbed labeled particle are liberated from the solid phase. Accordingly, photoacoustic spectrometry of a sample solution containing them results in causing serious measurement error, as a matter of course.

According to the method shown in Japanese Patent Application KOKAI No. 63-214668, it is unnecessary to use solid phase; the amount of signal detected from each of the labels becomes large, but washing is required using a centrifuging machine. Therefore, the operation is troublesome and automated only with difficulty. Further in Japanese Patent Application KOKAI No. 2-80937, no consideration is made to apply the method to immunoassay.

SUMMARY OF THE INVENTION

An object of the present invention is to provide a method for immunoassay which provides accurate results of measurements with high sensitivity even though an analyte is contained in a sample in a trace amount, and an apparatus for the immunoassay.

Another object of the present invention is to provide a method for immunoassay which can minimize as less as possible contamination of a particle label based on non-specific adsorption which did not bind to an analyte, and an apparatus for the immunoassay.

A further object of the present invention is to provide a method for immunoassay which can determine a plurality of analytes using a plurality of reactors having a reaction solid phase of the same mode, and an apparatus for the immunoassay.

The present invention relates to a method for immunoassay which comprises the steps of:

(a) supplying a sample to a reactor having a solid phase, to which a receptor is immobilized, to bind an analyte in the sample to the receptor;

(b) removing the unreacted sample from the reactor and then supplying a receptor labeled with fluorescent particles to the reactor to bind the labeled receptor to the solid phase via the analyte;

(c) removing an excess of the labeled receptor from the reactor and then supplying a liberating reagent containing a label-liberating agent to the reactor;

(d) introducing the fluorescent particle-containing solution to be assayed, which has been liberated from the solid phase, into a flow cell;

(e) detecting fluorescence based on the fluorescent particles which pass through the flow cell to count the number of the particles detected; and,

(f) computing the concentration of analyte in the sample based on the number of particles counted.

BRIEF DESCRIPTION OF THE DRAWINGS

Fig. 1 shows an outlined construction of one embodiment of the present invention.
Fig. 2 is a diagram for signal processing in two routes using the apparatus of Fig. 1.
Fig. 3 illustratively shows an embodiment of fixing or immobilizing a receptor onto a solid phase.
Fig. 4 illustratively explains the reaction state in a reactor.
Fig. 5 shows an example of calibration curve obtained using the apparatus of Fig. 1.
Fig. 6 shows an outlined construction of another embodiment of the present invention.
Fig. 7 is a drawing for explaining the procedure when CEA is measured with the apparatus of Fig. 6.
Fig. 8 shows comparison of the results when standard CEA sample was measured.

In the figures, numerals denote:
10
solid phase
11, 27
analyte (CEA)
15, 17
oligonucleotide
16, 26
receptor for solid phase (anti-CEA antibody)
21
fluorescent latex particle

23, 31
fluorescent particle
24, 24a, 29, 63
labeled antibody
25
restriction enzyme
32
turn table
33
sample container
35
reagent table
36a, 36b, 36c
container for labeled antibody solution
38
container for liberating reagent solution
42
movable arm
43
pipetting nozzle
46
controller
47
sheath flow cell
48
inlet for charging room
56
counter
61, 62
microplate
90a, 90b, 90c
container for antibody solution for fixing
94
tank for washing nozzle

DETAILED DESCRIPTION OF THE INVENTION

According to the present invention, the amount of a label associated with an antigen antibody reaction is counted as the number of the label, not in terms of its concentration. In order to avoid to count the labeled antibody non-specifically adsorbed to the solid phase, only the label bound by the antigen-antibody reaction is selectively liberated without liberating the non-specifically adsorbed labeled antibody, and only the liberated label is counted.

As the label in the present invention, fluorescent fine particles are preferably used. The method for immunoassay in accordance with the present invention comprises the steps of

supplying a sample to a reactor having a solid phase, to which a receptor is bound, to bind an analyte in the sample to the receptor;

removing the unreacted sample from the reactor, supplying a receptor labeled with fluorescent particles to the reactor to bind the labeled receptor to the solid phase via the analyte;

removing an excess of the labeled receptor from the reactor and then supplying a liberating reagent containing a label-liberating agent to the reactor;

introducing the fluorescent particle-containing solution to be assayed, which has been liberated from the solid phase, into a flow cell;

detecting fluorescence based on the fluorescent particles which pass through the flow cell to count the number of the particles detected; and

computing the concentration of analyte in the sample based on the number of particles counted.

A preferred embodiment of the present invention also comprises the following procedures, i.e., supplying a sample to a reactor having a solid phase, to which a receptor is bound via nucleic acid, polynucleotide or DNA, to immunologically bind an analyte in the sample to the receptor; supplying a labeled receptor to the reactor to bind the labeled receptor to the solid phase via the analyte; removing an excess of the labeled receptor from the reactor and then supplying a solution containing a restriction enzyme to the reactor to digest nucleic acid bound to the solid phase; introducing the label-containing solution to be assayed, which has been liberated from the solid phase, into a flow cell; and detecting the number of the label which passes through the flow cell.

As the receptor bound to the solid phase, where the analyte is an antigen or hapten, an antibody capable of specifically binding thereto is used; where the analyte is an antibody, an antigen capable of specifically binding to the antibody is used.

After a biological sample is supplied to the receptor with the solid phase to bind the analyte to the solid phase, a labeled antibody capable of immunologically binding to the analyte is added to the receptor. Thereafter, a solution containing an excess of the labeled antibody is removed from the receptor by suction, etc. By such operations for washing and removal, the labeled antibody non-specifically adsorbed to the surface of the solid phase is not split off but remains in the receptor.

Using the liberating reagent solution containing a specific label-liberating agent, only the antigen-antibody binding (immune complex) can be selectively dissociated, without substantially liberating (dissociating) the labeled antibody non-specifically adsorbed to the surface of the solid phase. Thus, only the label immunologically bound is selectively liberated into the solution in the receptor by supplying the liberating reagent solution. One of the specific label-liberating agent is a caotropic ion. As the caotropic ion, $CCl_3COO^-$, $SCN^-$, $CF_3COO^-$ and the like may be used.

Herein, nucleic acid for binding an antibody or antigen as the receptor to the solid phase includes oligonucleotide. The antibody or antigen on the solid phase in the receptor has been bound to the solid phase via double stranded nucleic acid, when a sample is supplied.

In the case of assaying for multiple items, many receptors having the same mode are prepared and as the analytical operations are initiated, specific receptors allotted to the respective analytes are bound to the solid phase. That is, single stranded nucleic acid having the same mode in the base sequence is previously fixed to the solid phase in each receptor and single stranded complementary nucleic acid having the receptors corresponding to various analytes is added to the reactors, as allotted, which are designed to correspond to the respective analytes, to form double strand (hybrid) on the solid phase in each reactor, where various receptors are bound. By doing to, a series of receptors for assaying multiple items which may immunologically react with various analytes are prepared.

After the analyte in the sample is immunologically bound to the solid phase in the reactor bound to the solid phase through nucleic acid, a labeled receptor is further bound thereto. In this case, a restriction enzyme is used as a label-liberating agent for splitting the label off from the solid phase. As the restriction enzyme, there may be advantageously used hydrolase such as restriction endonuclease, phosphodiesterase, pyrophosphatase, peptidase, esterase, etc. Many other known restriction enzymes may also be used. By such restriction enzymes, the double-stranded DNA is cleaved at a definite site.

The solid phase in the reactor comprises inner wall surface of the reactor itself, e.g., a test tube or a microplate, or spherical materials such as glass balls encased in the reactor, the surface of which has been so treated that can chemically bind to the receptor to be bound.

As the label or marker, fluorescent fine particles are advantageously used. The fluorescent particles are practically latex particles or inorganic particles, on the surface of which a fluorescent substance layer is formed. Alternatively, a mixture of particle-forming composition and fluorescent substance is particulated and the resulting particles may be practically used. Fluorescence is detected by a flow cyto-meter and the number of particles passed through the flow cell is counted. In this case, a diameter of the fluorescent particle is $10^{-5}$ to $10^{-3}$ mm. Examples of the fluorescent substance which can be used for the fluorescent particles are fluorescein, coumarine derivatives, rhodamine derivatives, dansyl, umbelliferone, etc. which are all known.

Where nucleic acid is cleaved and the label is liberated, the label-containing substance led to the flow cell is in such a state that a part of the cleaved nucleic acid, the receptor on the solid phase side, the analyte and the labeled receptor are combined with each other. On the other hand, nucleic acid bound to the receptor on the solid phase to which the analyte has not been bound is also cleaved with a restriction enzyme and led to the flow cell; however, since the label is not bound to such liberated matter, no fluorescence is detected in the flow cell so that measurement error does not occur substantially.

In the case of determining a concentration as in the prior art, the amount bound is counted as the statistical mean value. Therefore, detection sensitivity is insufficient at a low concentration and measurement data vary every sample by the influence of interferants contained in the sample so that accurate measurement data are not obtained. In addition, in order to avoid the influence of non-specific adsorption, it is attempted to perform the assay after dissociating the antibody-antibody complex; in this case, an additional problem encounters that a composition of the dissociation solution does not fit the conditions for enzyme reaction so that the dissociated sample cannot be measured as it is. By the procedures for dissociation and liberation, the solution to be assayed is diluted so that the lower limit for quantitative determination substantially goes up.

According to the method of counting the particles, even only one particle can be counted and hence, a detection sensitivity sufficient to measure an antigen of a low concentration can be obtained. That is, each particle gives an amplified signal. Using the particles as a label or marker, the number of the particles bound by antigen-antibody reaction is counted. Therefore, the count value directly reflects the amount of antigen.

According to the method of labeling fine particles and counting the particles one by one, it is designed to count a signal between two p.d. levels (high and low) on a predetermined definite level, even though interferants cover the surface of the particles to reduce or amplify the fluorescent intensity. By doing so, influence of the interferants can be avoided and the particles can be counted accurately. The counting is not affected by noise in the measurement system. Since the absolute number of the particles can be counted, there is no influence of dilution. The particle number can also be counted in the dissociation solution without modifying its composition. Therefore, no additional care is needed for measurement.

In the atmosphere, many dusts below several microns are present and these dusts contaminate the solution to be assayed, as foreign particles. In the method which comprises irradiating a sample with light and measuring the light scattered by the sample, the desired labeled particles are not distinguished from the foreign particles and the amount of foreign particles increases the background concentration. Turning to the method of the present invention for measuring fluorescence, foreign particles having a strong fluorescence are absent in normal environment and the measurement is thus made without any error. Therefore, detection can be made with high sensitivity by the method of the present invention for counting the particles one by one, using the fluorescent particles.

In nucleic acid, in the case of, e.g., DNA, constituent unit, deoxyribonucleotide is bound to 3',5'-phosphodiethyl ester. On the other hand, protein which constitutes the major part of an antibody is a polymer of amino acids by polypeptide bond. Since the binding mode of nucleic acid is quite dissimilar to that of protein, there is the least possibility that protein might be denatured or degraded under reaction conditions for digesting nucleic acid. In particular, when endonuclease called a restriction enzyme is used, the cleavage occurs only at a specific nucleotide sequence of nucleic acid. Therefore, only the nucleic acid portion can be selectively cleaved, without cleaving the protein portion in the conjugated matter.

Accordingly, an antibody is bound to the solid phase via nucleic acid, an antigen and a labeled antibody are captured by antigen-antibody reaction; after washing, selective release can be made at the nucleic acid portion. In this case, only the label participated in the desired antigen-antibody reaction can be split off from the solid phase, without denaturing or degrading the antibody or enzyme. The non-specifically adsorbed label not by the antigen-antibody reaction is directly adhered to the solid phase not via the nucleic acid. Such a label is not liberated by the action of restriction enzyme. Thus, the influence of non-specific adsorption is eliminated and the amount of antigen can be determined accurately. Quantitative nature is not damaged, either.

Among enzymes which digest DNA (deoxyribonucleic acid), a restriction enzyme functions to recognize only a specific nucleotide sequence and cleave the specific sequence. The nucleotide sequence is called recognition cleave sequence and inherent to each restriction enzyme. Accordingly, where the recognition cleavage sequence of restriction enzyme used is inserted at at least one site in the nucleotide sequence of polynucleotide or oligonucleotide used for the binding, the restriction enzyme can be acted thereon to effect cleavage. The nucleotide sequence satisfying the condition can be freely set forth.

A reactor having a solid phase, to which a receptor such as an antibody is bound, is extremely advantageous for an immunoassay as stated hereinbefore, and therefore may be widely used for various immunoassays. That is, the reactor may be also used for a conventional immunoassay wherein an enzyme is used as a label or wherein instead of flow cell, a label is detected by conventionally measuring an optical absorbance. Thus, according to the other aspects of the present invention, there is also provided a method for immunoassay which comprises the steps of; supplying a sample to a reactor having a solid phase, to which a receptor is bound via nucleic acid, to bing an analyte in the sample to the receptor; removing the unreacted sample from the reactor and then supplying a label to the reactor to bind the label to the solid phase via the analyte; removing an excess of the label from the reactor and then supplying a restriction enzyme to cleave the nucleic acid; and detecting the label which has been liberated from the solid phase.

In the above method, as the label, there may be used an enzyme such as a peroxidase, and a fluorescence substance such as fluorescein and coumarine derivatives. The liberated label may be detected by utilizing an enzyme reaction. A conventional procedure may be used in the above immunoassay, as it is.

As a kit for the above immunoassay, there is provided a kit comprising a reactor having a solid phase, to which a receptor is bound via nucleic acid; and a restriction enzyme.

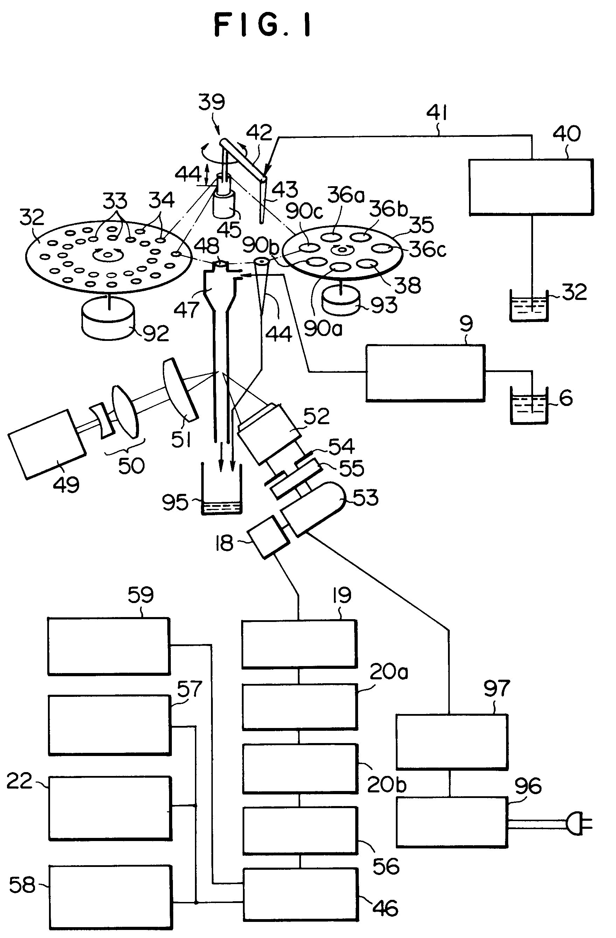

The first embodiment of the present invention is described by referring to Figs. 1 to 5.

Fig. 1 shows an outline of the entire construction of the apparatus for immunoassay which is an embodiment of the present invention. A series of containers may be placed in two lines on turn table 32. A plurality of sample containers 33 encasing blood samples are circularly arranged at the inner circumference and a plurality of reactors 34 each having a solid phase are circularly arranged at the outer circumference. As later described, single stranded complementary oligonucleotide is fixed on the solid phase of each reactor 34. The turn table 32 is rotated by pulse motor 92 operatively controlled by controller 46 to stop the desired sample container 33 and reactor 34 at the position of charging through pipetting nozzle 43. The line of the reactors is constructed to move in a thermostat.

A variety of reagent containers necessary for analytical operations are placed on reagent table 35. That is, containers 90a, 90b and 90c in which solutions of antibodies for fixing corresponding to various items A, B and C for analysis are charged, containers 36a, 36b and 36c in which solutions of fluorescent particle-labeled antibodies corresponding to the various items for analysis are charged, container 38 in which a liberating reagent solution containing a restriction enzyme is charged, and other necessary buffer containers, etc. are held on reagent table 35. The pulse motor 93 operatively controlled by controller 46 rotates reagent table 35 by a desired angle and positions the instructed reagent container at the position of sucking through nozzle 43, with a necessary timing.

Automated pipetting mechanism 39 is equipped with pipetting nozzle 43 mounted to movable arm 42, rotary driving part 45 for horizontally rotating arm 42, vertical driving part 44 for vertically moving arm 42, syringe pump 40 connected with nozzle 43 via tube 41, and washing solution tank 37 which also acts as an extrusion solution. With the movement of arm 42, pipetting nozzle 43 can rotate on the two charging positions on turn table 32, the position of receiving reagent on reagent table 35, inlet 48 for room of flow cell 47 and nozzle washing tank 94, as a rotary radius. The nozzle can go down and go up at the respective positions.

The inside of sheath flow cell 47 is constructed as in that used for known flow sight meter, but a reagent charging room equivalent to that shown in Japanese Patent Application KOKAI No. 2-80937 is opened at the upper portion. Therefore, nozzle 43 can enter into charging room inlet 48 and discharge the solution to be assayed into sheath flow cell 47. By feeding pump 9, the sheath solution in sheath solution tank 6 is fed at a constant flow amount, flows along the inner wall of flow cell 47 and discharged in discharge reservoir 95. The solution to be assayed which is introduced into the flow cell flows at the center of the flow of the sheath solution.

A laser light source 49 can emit an argon laser having an oscillation wavelength of 488 nm. After its beam width is expanded by beam expander 50, a flux of the laser light is irradiated on sheath flow cell 47 to stop down by lens 51 and focus on the flow of the solution to be assayed. For condensing the fluorescence from flow cell 47, objective lens 52 for microscope is used. Space filter 54 and wavelength selection filter 55 are provided in front of photomultiplier 53 as a photoelectric detector to remove scattered light and Raman scattering light. After the output of photomultiplier 53 is amplified by preamplifier 18, the output is amplified by linear amplifier 19 and noise is removed by lower pulse height analyzer 20a and upper pulse height analyzer 20b. Thereafter, the pulse between the two threshold values is integrated.

A high voltage is applied to photomultiplier 53 via transducer 96 and high voltage electric source 97. Sample numbers, results of counting, calibration curve, histogram for measuring fluorescence, etc. are output on display 57, printer 22 and floppy disk 58. Also via interface 59, they may be communicated with a personal computer.

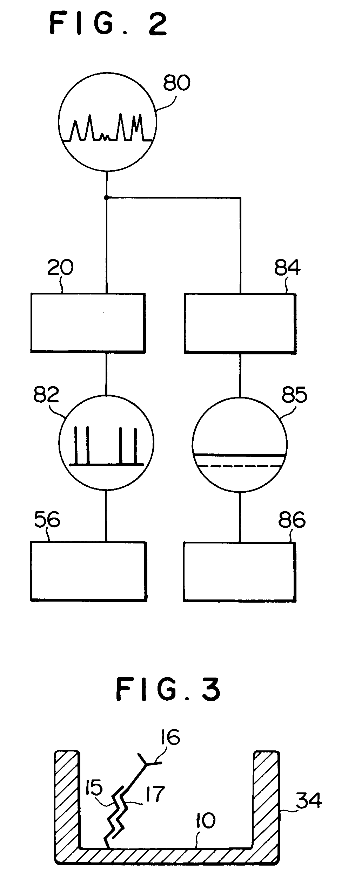

A diagram for signal processing is shown in Fig. 2. Via circuit 20 for analyzing the output 80 of preamplifier by pulse height analyzer, pulse train 82 is obtained. The pulse train is integrated with counter 56. In another circuit for signal processing, output 80 is passed through levelling circuit 84 to convert the pulse number into voltage change to obtain signal 85. The voltage change is counted with volt meter 86. The counting with this levelling may also be conducted by connecting with, e.g., a pen recorder and reading the recorded value.

Examples of the items determined by the apparatus shown in Fig. 1 are carcinoembryonic antigen (CEA, C-reactive protein (CRP) and $\alpha$-fetoprotein (AFP). In this case, a solution containing anti-CEA antibody bound to single stranded oligonucleotide is charged in container 90a for antibody solution for fixing on reagent table 35; a solution containing anti-CRP antibody similarly bound is charged in container 90b for antibody solution for fixing; and, a solution containing anti-AFP antibody similarly bound is charged in container 90c for antibody solution for fixing. In containers 36a, 36b and 36c for labeled antibody solution, anti-CRP antibody, anti-digoxin antibody and anti-AFP antibody labeled with fluorescent fine particle, respectively, are charged.

The process of immuno-binding in each item for assay is similar; therefore, taking the case of CEA as an example, the process for analytical operations in the reactor is explained hereafter. The reactors 34 on turn table 32 all take the same mode prior to the procedures for assay. That is, as shown in Fig. 3, the inner wall of glass-made reactors 34 forms solid phase 10 and single stranded oligonucleotide 15 is fixed to the solid phase. The items for analysis are specified by the kind of antibody for fixing which is charged after the operation has started. Therefore, the same oligonucleotide 15 is fixed to each of the reactors 34 prior to initiation of the operations. Oligonucleotide 15 is bound to the solid phase having an amino group as the surface active group.

On the other hand, a solution containing antibody for fixing, in which anti-CEA antibody is bound to synthetic oligonucleotide having nucleotide sequence AGGCCT by maleimide thiol coupling and the sequence can be cleaved with restriction enzyme HaeI, is prepared, wherein, A, G, T and C are constituent units of nucleotide and denote adenine, guanine, thymine, and cytosine, respectively.

When the procedures for analysis are initiated, the antibody is dispensed from container 90a for antibody solution through nozzle 43. By discharging in the corresponding reactor 34, it is specified that the reactor is for assaying CEA. As shown in Fig. 3, oligonucleotide 17 in anti-CEA antibody 16 is bound to oligonucleotide 15 having the complementary strand in the solid phase side. Accordingly, this reactor 34 can recognize CEA.

Where the thus treated reactor is prepared prior to initiation of the operations for analysis, the reactor may be the one having a reactive solid phase capable of recognizing each item for analysis. Also for specifying other items, such can be effected simply by changing the solution of antibody for fixing.

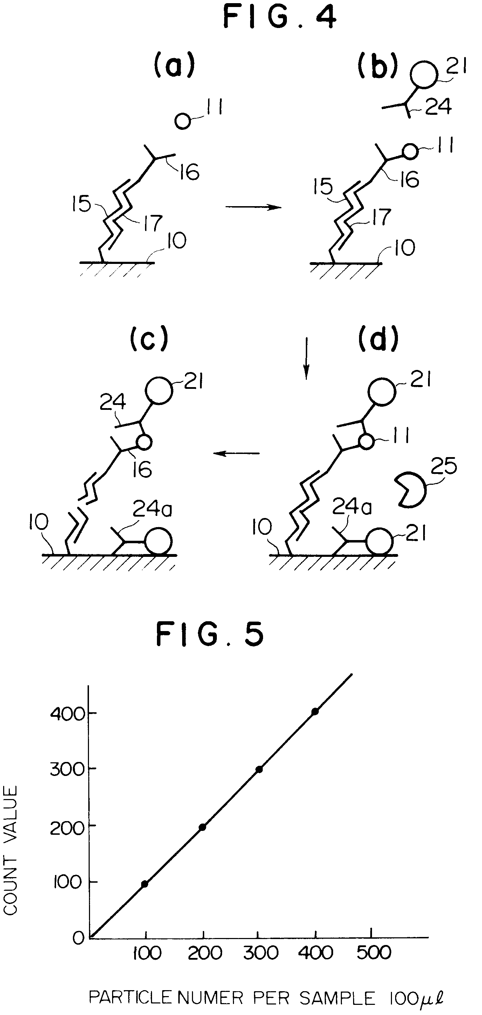

In the thus prepared reactor 34, a sample dispensed from sample container 33 through nozzle 43 is charged. As shown in Fig. 4 (a), CEA 11 in the sample is bound to anti-CEA antibody 16 fixed onto solid phase 10. After reacting for 10 minutes, the unreacted sample from reactor 34 is sacked through nozzle 43 and discharged to washing tank 94. Then, buffer is added to reactor 34 followed by sucking and discharging through nozzle 43. This washing operation is repeated several times.

Subsequently, as shown in Fig. 4 (b), a solution containing anti-CEA antibody labeled with latex particles 21 which contain a coumarine derivative as a fluorescent substance is sucked from container 36a on reagent table 36a in a definite volume through nozzle 43 and discharged into the corresponding reactor 34. The reactor 34 is maintained at a definite temperature (37°C) for a definite time period (15 minutes) on the turn table to perform the reaction, whereby labeled anti-CEA antibody 24 is bound to CEA 11 and fixed onto solid phase 10. In this case, as shown in Fig. 4 (c), a part of labeled anti-CEA antibody 24a which was not immunologically bound to CEA 11 is directly adsorbed onto the surface of solid phase 10. The solution containing an excess of the labeled antibody in reactor 34 is sucked and discharged into washing tank 94, through nozzle 43. Thereafter, the washing solution is discharged into the reactor through nozzle 43 and the operation for sucking and discharging the washing solution is further repeated.

Then, container 38 for liberating reagent solution is located at the sucking position by rotating reagent table 35. The solution containing restriction enzyme Hae I 25 in the container 38 is sucked in a definite volume through nozzle 43 and discharged into the corresponding reactor 34 on turn table 32. As shown in Fig. 4 (c) and (d), the double-stranded oligonucleotide bound to solid phase 10 is cleaved at the digesting site in the reactor and the liberated substance is suspended in the solution in reactor 34. Also for such liberation, a definite period of time (15 minutes) is required. By cleaving the specific hybrids of complementary nucleic acid or double-stranded DNA in the middle with restriction enzyme 25, the two liberated substances are present in the reactor. One of them is the fluorometrically detectable liberated matter composed of antibody 24 with label 21, antigen 11, antibody 16 for fixing and restricted fragment of oligonucleotide, and another is the fluorometrically non-detectable isolated matter composed of the antibody for fixing which remains on the solid phase without causing immunological binding, and restricted fragment. The solution to be assayed which contain these liberated matters is sucked through pipetting nozzle 43 and discharged into sheath flow cell 47. Upon liberation with restriction enzyme 25, labeled antibody 24a directly adsorbed onto solid phase 10 is not liberated so that such does not contaminate the solution to be assayed.

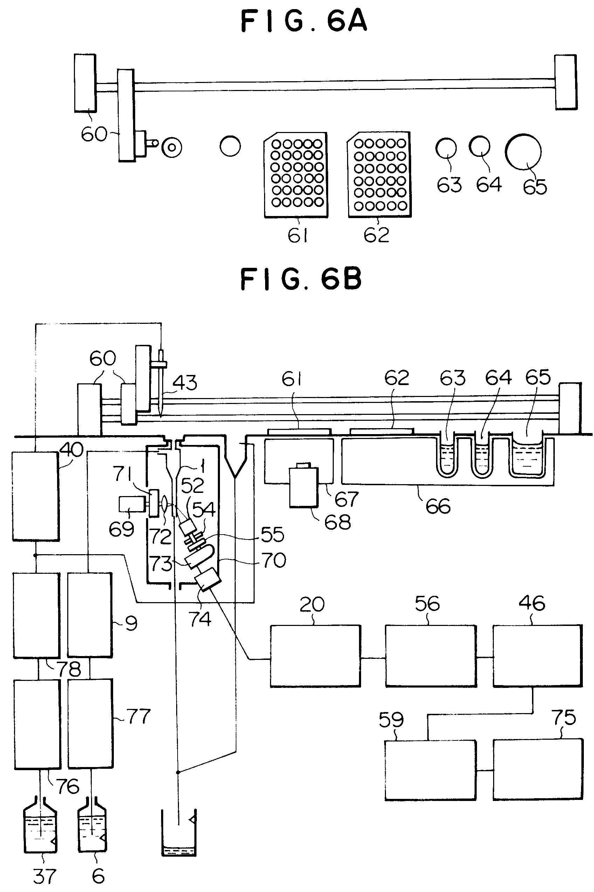

By using as latex particles 21 containing a coumarine derivative the particles having a particle diameter of 0.1 $\mu$m and CEA standard sample instead of the sample described above, a calibration curve is obtained and shown in Fig. 5. The calibration curve has high linearity for quantitative determination and is excellent especially for quantitative determination at a low concentration. No curve observed with the assay using enzyme reaction at a low concentration was noted in this case. The lower limit of detection was determined by contrasting one particle to one molecule; it was below $10^{-22}$ mol as the absolute amount and below $10^{-18}$ mol/l as the concentration.

In the apparatus shown in Fig. 1, even in the case of assaying for a plurality of items, reactor 34 placed on turn table 32 may be the one equipped with the solid phase of the same mode. When the relationship between a sample and items to be assayed is input from an input device to controller (computer) 46, controller 46 controls the operation for each part of the apparatus to perform measurement of necessary items in a desired order. For example, where items are desired to be assayed in the order of A, A, B, C and B, the antibody for fixing is charged in each reactor in the order of 90a, 90a, 90b, 90c and 90b and fixed. The controller 46 also controls dispersed samples to perform measurement in an optional order. A sample is diluted, if necessary and desired.

After the sample is reacted, reactor 34 is washed and labeled antibodies 36a, 36b and 36c are then dispensed. The labeled antibodies are those obtained by binding labels to antibodies corresponding to the items A, B, and C to be assayed and therefore, the labeled antibodies are also automatically chosen by controller 46 in the order of 36a, 36a, 36b, 36c and 36b, according to the order of A, A, B, C and B initially designated.

Next, a second embodiment of the present invention is explained by referring to Figs. 6 to 8. In the apparatus for immunoassay shown in Fig. 6, the inner wall of microcapsule is used as the solid phase and X, Y moving mechanism is used in place of the turn table in the first embodiment.

In Fig. 6, microtiterplate 61 for reaction, microtiterplate 62 for sample, labeled antibody 63, liberating solution 64 and washing solution 65 are placed on the upper surface of the apparatus. The reagent part is kept cool with cooler 66. The reaction part is kept at 37°C with heater 67. The reaction part and labeled antibody 63 are designed to be shaken with vibrator 68.

As light source 69, He-Ne laser light having an oscillation wavelength of 632.8 nm is used. A light shielding plate 70 is provided so as to avoid incident light from the outside.

Laser light is shaped through prism 71 and condensed by lens 72. Fluorescence is condensed by lens 52, passed through spatial filter 54 and interference filter 55, and then detected with semiconductor optical detector 73. After amplifying with amplifier 74 and selecting the signal with pulse height analyzer 20, the signal is input into counter 56. Data processing is conducted with computer 46, which is connected with interface 59 to also communicate with outer equipments.

IN addition to a printer, output is also made into IC memory card 75. Initial setting of the apparatus is also memorized on the IC card to make it easy to start.

As the fluorescent fine particles, particles having a diameter of 0.1 and 0.2 $\mu$m containing methylene blue are used.

In order to avoid adverse affect of bubble contained in the sheath solution, etc., deaerators 76 and 77 are mounted.

For feeding the sheath solution and the solution to be assayed, a pneumatic pump may be constructed by pressurized air and such may be used for this purpose, instead of syringe pump 40. In this case, there is an effect of reducing pulse flow.

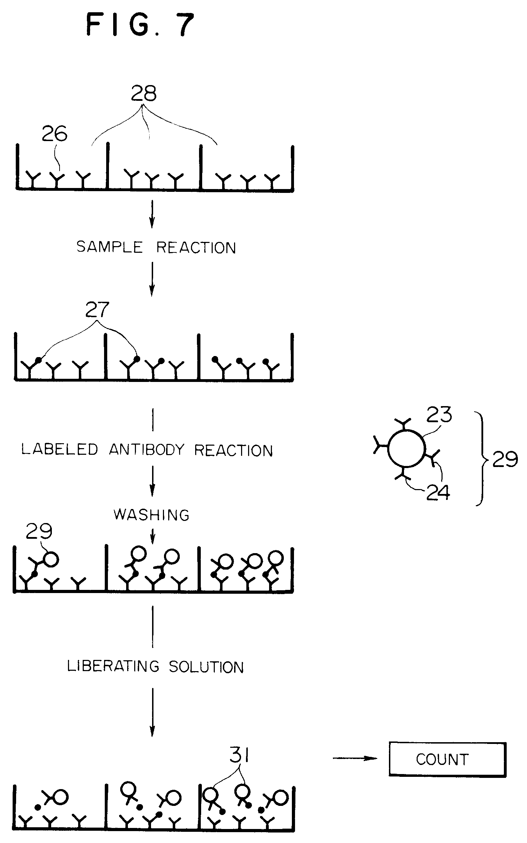

The labeled antibody obtained by binding an antibody to commercially available fluorescent fine particles is prepared and CEA is immunologically assayed, using the apparatus shown in Fig. 6. This embodiment is explained by referring to the illustrated drawing of Fig. 7.

Anti-CEA antibody is bound to fluorescent particles 23 (containing fluorescein; particle diameter of 0.1 $\mu$m, Molecular Probe Inc.), on the surface of which carboxy groups are introduced, using water-soluble carbodiimide.

Anti-CEA antibody 26 is bound to polystyrene-made microplate 61, which is used as the reactive solid phase.

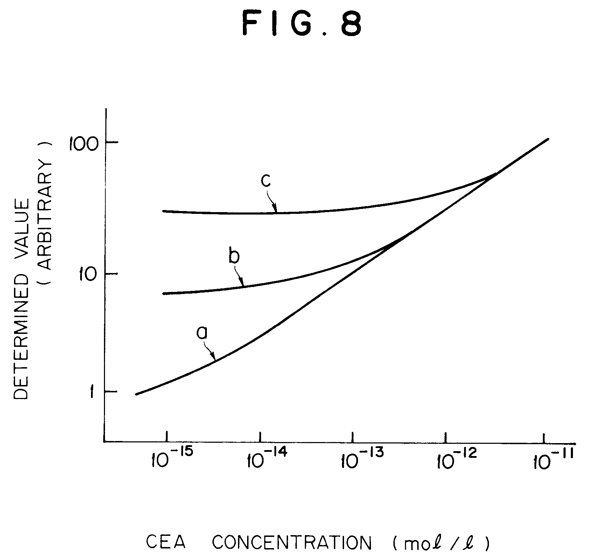

Prior to the operations for measurement, CEA standard solution is prepared, which is made sample 27. Sample 27 is added to each well 28 of microplate 61 fixed with anti-CEA antibody 26, which is allowed to stand at room temperature for a definite period of time. After washing, labeled antibody 29 labeled with fluorescent particles 23 is added thereto to react them for a definite period of time. After thoroughly washing, a caotropic liberating solution 64 is added to liberate the fluorescent particles 31 captured onto the microtiterplate by antigen-antibody reaction. The solution containing the liberated fluorescent particles is made a solution to be assayed and the number of fine particles in the solution is counted. A calibration curve for CEA concentration is shown in Fig. 8, a.

For purpose of comparison, a calibration curve is prepared using as a label horse radish peroxidase (POD) which is one of enzyme, in the method for detecting the amount of POD by reacting with a fluorescent substrate. In this case, the label (POD) is liberated after the immune reaction, transferred to a separate microplate and subjected to enzyme reaction (shown by b. in Fig. 8). In addition, the label is not liberated but subjected to enzyme reaction in the same microplate as it is and then fluorometrically measured (shown by c. in Fig. 8).

It is obviously noted that the results a. according to the process of the present invention provide a lower background concentration and higher detection sensitivity at a low concentration.

In the fine particle counter explained in Embodiment 1, attention is paid only to the pulse component of the signal obtained by photoelectric amplifier, and this pulse number is counted. With a sample having a low density in the particle number, measurement data showing extremely high quantitative nature are obtained, as has already been explained. However, with a sample having a high density of the particle number, more than 2 particles pass across the flux of laser light which might result in failing to count and thus reduction in counting rate. On the other hand, with a sample of high concentration, the fluorescent intensity becomes strong so that the fluorescent intensity namely, the signal may be levelled with a definite time constant; even if its series component is counted, the measurement value in proportion to the concentration is given. Accordingly, if both the pulse number and the series component value are simultaneously measured, the region of concentration to be assayed can be broadened. By adopting such a process, the sensitivity of immunoassay can be increased by about two figures.

According to the present invention, accurate measurement results with high sensitivity can be obtained even though the analyte is contained in a sample in a trace amount. Therefore, the present invention greatly contributes to immunoassay.

**Claims**

1. A method for immunoassay which comprises the steps of:
   (a) supplying a sample to a reactor having a solid phase, to which a receptor is bound, to bind an analyte in the sample to the receptor;
   (b) removing the unreacted sample from the reactor and then supplying a receptor labeled with fluorescent particles to the reactor to bind the labeled receptor to the solid phase via the analyte;
   (c) removing an excess of the labeled receptor from the reactor and then supplying a liberating reagent containing a label-liberating agent to the reactor;
   (d) introducing the fluorescent particle-containing solution to be assayed, which has been liberated from the solid phase, into a flow cell;
   (e) detecting fluorescence based on the fluorescent particles which pass through the flow cell to count the number of the particles detected; and,
   (f) computing the concentration of analyte in the sample based on the number of particles counted.

2. A method for immunoassay according to claim 1, wherein said receptor is an antibody and said analyte is an antigen or hapten.

3. A method for immunoassay according to claim 1, wherein said fluorescent particles are latex particles or inorganic particles, on the surface of which a fluorescent substance layer is formed, or particulated a mixture of particle-forming composition and a fluorescent substance.

4. A method for immunoassay according to claim 1, wherein the particle diameter of said fluorescent particle is $10^{-5}$ to $10^{-3}$ mm.

5. A method for immunoassay according to claim 1, wherein said liberating agent contains caotropic ions.

6. An apparatus for immunoassay which performs operations for immunoassay according to claim 1.

7. A method for immunoassay which comprises the steps of:
   (a) supplying a sample to a reactor having a solid phase, to which a receptor is bound via nucleic acid, to immunologically bind an analyte in the sample to the receptor;
   (b) supplying a labeled receptor to the reactor to bind the labeled receptor to the solid phase via the analyte;

(c) removing an excess of the labeled receptor from the reactor and then supplying a solution containing a restriction enzyme to the reactor to cleave nucleic acid bound to the solid phase;

(d) introducing the label-containing solution to be assayed, which has been liberated from the solid phase, into a flow cell; and

(e) detecting the number of the label which passes through the flow cell.

8. A method for immunoassay according to claim 7, wherein said nucleic acid has double-stranded DNA, or specific hybrids of complementary nucleic acid.

9. A method for immunoassay according to claim 7, wherein said nucleic acid is oligonucleotide.

10. A method for immunoassay according to claim 7, wherein said solid phase comprises a spherical substance encased in said reactor or the inner wall of said reactor, which is subjected to surface treatment that is chemically bound to said receptor.

11. An apparatus for immunoassay for which performs operations for immunoassay according to claim 7.

12. A method for immunoassay which comprises the steps of:

(a) preparing a plurality of reactors having a solid phase to which one of single-stranded nucleic acid capable of forming a hybrid is bound;

(b) supplying a first receptor to which another single-stranded nucleic acid capable of complementarily binding to said one single-stranded nucleic acid is bound to a first reactor among said plurality of reactors, wherein said first receptor is capable of specifically binding to a first analyte in a sample;

(c) supplying a second receptor to which the same as another single-stranded nucleic acid is bound and which is capable of specifically binding to a second analyte to a second reactor amount said plurality of reactors; and

(d) supplying a sample to said first and second reactors to proceed the immunoreaction of said first analyte in said first reactor and proceed the immunoreaction of said second analyte in said second reactor.

13. A method for immunoassay according to claim 12, characterized by supplying a labeled receptor to said reactors after the antigen-antibody reaction, removing an excess of the labeled receptor from said reactors and then supplying a solution containing a restriction enzyme to said reactors, and introducing the solution containing the label liberated from said solid phase into a flow cell as a solution to be assayed.

14. An apparatus for immunoassay for performing a method for immunoassay according to claim 13.

15. A method for immunoassay which comprises the steps of:

supplying a sample to a reactor having a solid phase, to which a receptor is bound via nucleic acid, to bind an analyte in the sample to the receptor;

removing the unreacted sample from the reactor and then supplying a label to the reactor to bind the label to the solid phase via the analyte;

removing an excess of the label from the reactor and then supplying a restriction enzyme to cleave the nucleic acid; and

detecting the label which has been liberated from the solid phase.

16. A method for immunoassay according to claim 15, which comprises the steps of:

binding a particle label to a solid phase in a reactor via nucleic acid;

supplying a restrction enzyme to said reactor to cleave said nucleic acid; and

introducing into a flow cell the particle label which binds a part of nucleic acid after said cleavage.

17. A reactor having a solid phase at the inner wall thereof, in which a receptor is bound to said solid phase via nucleic acid.

18. A kit for immunoassay which comprises:
a reactor according to claim 17; and
a restriction enzyme.

# FIG. 1

# FIG. 2

# FIG. 3

# F I G . 4

## (a)

## (b)

## (c)

## (d)

# F I G . 5

PARTICLE NUMER PER SAMPLE 100μℓ

# FIG. 6A

# FIG. 6B

# FIG. 7

SAMPLE REACTION

LABELED ANTIBODY REACTION

WASHING

LIBERATING SOLUTION

COUNT

# F I G . 8

CEA  CONCENTRATION  ( mol / l )